# FASCICULE DE BREVET EUROPEEN

(11) **EP 0 595 743 B1**
(45) Date de publication et mention de la délivrance du brevet: **14.03.2001**
(21) Numéro de dépôt: 93430015.3
(22) Date de dépôt: 26.10.1993
(51) Int. Cl.: C07K 7/02, A61K 38/08, A61K 39/385

(54) **Dérivés bi-haptènes liant le technétium ou le rhénium, procédé de préparation, application diagnostic et thérapeutique, kits et réactifs immunologiques.**
Verbindungen mit zwei Haptenen zur Bindung von Technetium oder Rhenium, ihr Herstellungsverfahren, ihre Anwendung in Diagnostik und Therapie und sie enthaltende Reagenziensätze und immunologische Reagenzien.
Bihaptinic derivatives for binding technetium or rhenium, process for their preparation, application for diagnosis and therapy, kits and immunological reactants comprising them.

(30) Priorité: 27.10.1992 FR 9213267
(43) Date de publication de la demande: 04.05.1994
(73) Titulaire: IMMUNOTECH PARTNERS: Société Anonyme dite, F-13276 MARSEILLE CEDEX 9 (FR)
(72) Inventeur: Gruaz-Guyon, Anne, F-92100 Boulogne (FR); Le Doussal, Jean-Marc, F-13009 Marseille (FR); Delaage, Michel, F-13001 Marseille (FR); Barbet, Jacques, F-13008 Marseille (FR)
(74) Mandataire: Rinuy, Santarelli

(56) Documents cités:
- EP-A- 0 419 387
- BIOCONJUGATE CHEMISTRY vol. 1, no. 6 , Novembre 1990 pages 431 - 437 R.W. WEBER ET AL. 'Enhanced Kidney Clearance with an Ester-Linked 99mTc-Radiolabeled Antibody Fab'-Chelator Conjugate'

## Description

La présente demande concerne de nouveaux dérivés bi-haptènes liant le technétium ou le rhénium, leur procédé de préparation, leur application à la thérapie ou au diagnostic, les kits et réactifs immunologiques les renfermant.

La demande de brevet Européen 0 173 424 décrit des chélates de Technétium radiomarqué, et leur utilisation dans l'exploration des fonctions rénales.

Bioconjugate Chemistry, vol. 1, 1990, pages 431-437 enseigne le couplage de chélateurs bifonctionnels basés sur le noyau N3S de la mercaptoacétyltriglycine et possédant un groupement ester ou amide, sur des groupes sulfhydriles d'un fragment Fab' d'anticorps antimyosine.

Les demandes de brevet européen N° 0 263 046 et 0 419 387 ont décrit des immuno-réactifs destinés à cibler les cellules animales pour leur visualisation ou leur destruction in vivo.

Ces immuno-réactifs consistent en deux types de produits à savoir :
a) un anticorps monoclonal ou un fragment d'anticorps reconnaissant un type cellulaire, un tissu particulier ou un constituant d'un organisme animal conjugué à un second anticorps ou fragment d'anticorps reconnaissant un groupement hapténique déterminé et
b) une molécule constituée d'au moins deux haptènes identiques ou différents reconnus par l'un des anticorps et d'un motif constitué d'un élément de visualisation, ou d'un produit actif ou connu pour pouvoir recevoir un élément de visualisation ou un produit actif destiné à la destruction de la cellule-cible.

Dans ce qui suit, on appellera le produit (b) "sonde". "Groupement hapténique" et "haptène" seront utilisés indifféremment pour désigner la même entité chimique. On appellera "cible" le type cellulaire ou cellule, le tissu particulier ou le constituant d'un organisme vivant que l'on souhaite localiser, visualiser ou détruire. De même, on appellera indifféremment "conjugué bispécifique" ou "anticorps-bispécifique" le produit (a).

Lorsque l'on veut réaliser une bonne visualisation d'une cellule-cible, il est souhaitable de réduire au maximum le bruit de fond. De même, étant donné que les produits radiothérapeutiques ou de radiodiagnostic possèdent habituellement une certaine agressivité, il est souhaitable de les retrouver seulement au niveau de la cible, le plus rapidement après leur administration.

Les sondes précédemment décrites peuvent être marquées efficacement par différents isotopes radioactifs, particulièrement l'indium 111, l'iode 123 ou l'iode 131 pour la visualisation de la cible et l'iode 131 pour la destruction des cellules cibles.

Toutefois, l'indium est un métal coûteux. L'iode 123, pour sa part est d'un approvisionnement difficile. De plus certains de ces produits tels l'Iode 131 sont des isotopes à durée de vie longue.

Il serait donc souhaitable, de disposer de sondes très spécifiques, efficaces, capables de fixer des isotopes bon marché, d'approvisionnement aisé, ainsi que d'une durée de vie courte afin de permettre une administration à des doses plus élevées tout en permettant une durée d'exposition plus courte des corps, notamment humains, aux radiations.

C'est pourquoi la présente demande a pour objet des sondes (b) comprenant deux haptènes hydrophiles et un groupement potentiellement effecteur caractérisées en ce qu'elles répondent à la formule générale 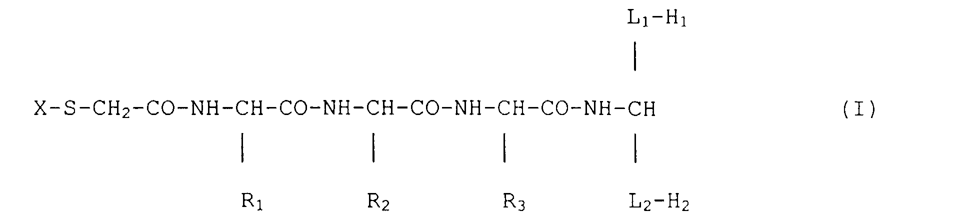 ainsi que leurs sels, dans laquelle X représente un atome d'hydrogène ou un groupe protecteur utilisable pour protéger une fonction thiol contre l'oxydation, R₁, R₂ et R₃ représentent chacun une chaîne latérale d'acide aminé, L₁ et L₂ représentent chacun une liaison stable permettant la fixation simultanée sur la même molécule sonde d'au moins 2 anticorps spécifiques sur les haptènes, la distance séparant les haptènes étant supérieure à 10 Angströms et H₁ et H₂ représentent des haptènes hydrophiles. Les haptènes H₁ et H₂ peuvent être identiques ou différents. Dans la formule I, le groupement 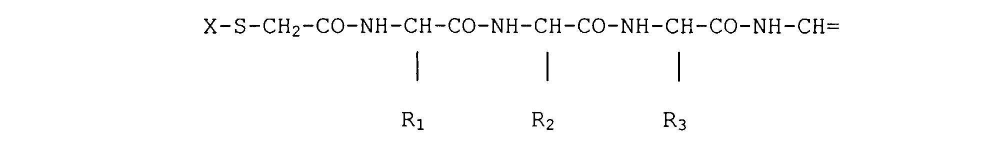 représente le groupement potentiellement effecteur.

Le groupe protecteur utilisable pour protéger une fonction thiol contre l'oxydation peut être par exemple un radical CH₃-CO-NH-CH₂-, CF₃CO-, ou HO-CH₂-CO-, et de préférence un radical CH₃CO- ou C₆H₅CO-.

Les acides aminés peuvent être choisis de préférence parmi les acides aminés naturels, de séries D ou L, et notamment parmi le groupe constitué de la glycine, de l'acide aspartique, de l'acide glutamique, de l'aspargine, de la glutamine et de la lysine. Notamment R₁, R₂ et R₃ sont identiques et représentent particulièrement un atome d'hydrogène.

Les sels des dérivés de formule I peuvent être obtenus par réaction avec une base ou un acide de façon à assurer une solubilité suffisante dans l'eau et à ramener le pH de la solution à une valeur comprise entre 5 et 9. Ils doivent être compatibles avec l'activité chélatante du groupement potentiellement effecteur. On préfère utiliser des bases ou des acides connus pour former des sels peu ou pas toxiques. On utilise de préférence des chlorhydrates, acétates, citrates, tartrates ou des sels de sodium et ammonium.

Par "haptène", l'on entend dans le cadre de la présente invention, une molécule capable de se lier spécifiquement à un anticorps au niveau d'un de ses sites de fixation. C'est une molécule qui n'est pas antigénique par elle-même mais peut le devenir par fixation sur une macromolécule. Sa masse moléculaire est généralement inférieure à 1500 daltons.

Les haptènes préférés selon la présente invention auront une masse moléculaire comprise entre 300 et 1500 daltons et comporteront des groupements chimiques polaires. Ces groupements seront notamment des groupes alcools, amines, acides carboxyliques, sulfoniques et phosphoriques, ainsi que leurs esters et amides.

On peut trouver des exemples de haptènes notemment dans "Methods in Enzymology Vol 84, J.Langone and H. Van Vunakis Ed. Academic Press, New York 1982.

Parmi les dérivés objet de la présente invention, on retient de préférence ceux caractérisés en ce que les haptènes ont une constante de dissociation inférieure ou égale à 10⁻⁵ M et notamment inférieure ou égale à 10⁻⁶ M avec un anticorps qui leur est spécifique. Cette constante est mesurée dans les conditions usuelles de pH, température et concentration en sels.

Comme indiqué dans les demandes de brevet précitées, les haptènes peuvent revêtir une grande variété de structures.

A titre de haptènes, on retient également les dérivés des acides, amines ou aminoacides polaires liés de façon covalente par des liaisons amides et particulièrement de l'histidine et les dérivés de l'histamine, tout particulièrement de l'histamine-succinyl-glycine. Ces composés comportent de préférence de 3 à 6 amines, acides ou acides aminés et notamment 3 ou 4 acides aminés, notamment polaires, liés de manière covalente de préférence par des liaisons amide.

Parmi les haptènes H₁ et H₂ ci-dessus, on retiendra aussi les produits correspondant à la formule :

A-CO-(CH₂)₂-CO-Y-

dans laquelle A représente un composé aminé polaire tel que, par exemple, l'histamine, la 5-hydroxy-tryptamine, la tyramine, la 3-iodo-4-hydroxy-phenyl éthylamine et Y représente un acide aminé choisi dans le groupe constitué par la glycine, l'alanine, l'acide aspartique, l'acide glutamique, la tyrosine, l'asparagine ou la glutamine, ces acides aminés pouvant être de la série L ou D. Les liaisons entre A et Y et l'acide succinique central sont des liaisons peptidiques.

D'autres haptènes également préférés sont décrits dans la formule suivante :

B-NH-CH₂-CO-Y-

dans laquelle B représente un composé acide polaire tel que par exemple, les acides imidazolyl acétique, 5-hydroxy-indole-acétique, 4-hydroxyphenyl-acétique, 3-iodo-4-hydroxy-phényl acétique et Y correspond à un amino acide choisi dans le groupe constitué par la glycine, l'alanine, l'acide aspartique, l'acide glutamique, la tyrosine, l'asparagine ou la glutamine, ces acides aminés pouvant être de la série L ou D. Les liaisons entre B et Y et l'acide succinique central sont des liaisons peptidiques.

Par haptènes "hydrophiles", l'on entend que le coefficient de partition entre une phase aqueuse neutre (proche des conditions physiologiques) et un solvant organique non totalement miscible à l'eau comme le N-butanol est supérieur à 1 pour le haptène isolé. Cette mesure peut être effectuée, notamment après deux heures d'agitation d'une solution d'haptène dans 10mM Hepes, 150mM NaCl, pH 7,4 mélangée à une partie égale de butanol.

Dans une sonde, les haptènes et le(s) groupe(s) effecteur(s) sont reliés de manière stable directement ou par une chaîne de connexion. Celle-ci permet la fixation simultanée sur une même molécule sonde d'au moins deux anticorps spécifiques sur le ou les haptènes et ainsi la formation des complexes particuliers recherchés. La liaison sera assez courte pour ne pas altérer la solubilité dans l'eau, ni permettre la liaison avec deux sites du même anticorps. Elle sera assez longue pour permettre la liaison de deux anticorps différents. Typiquement, la distance entre les haptènes sera de préférence comprise entre 10 et 80 Angströms.

L'on entend par "groupement effecteur", un groupement chimique responsable de l'effet désiré, consécutivement à l'utilisation de la sonde, sur sa cible dans l'organisme. Le groupement effecteur peut réaliser soit le repérage des sites d'accumulation de la sonde, soit par exemple la destruction de cellules au niveau de sites d'accumulation. Dans le cadre de la présente demande, la chaîne 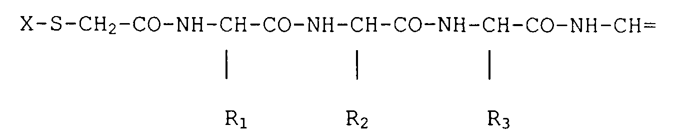 est le groupement potentiellement effecteur du dérivé de la formule I.

Dans le premier type d'application, le groupement potentiellement effecteur pourra fixer par exemple un atome repérable par des moyens physiques appropriés, comme par exemple des atomes radioactifs émetteurs de rayonnement, notamment gamma, particulièrement le Technetium -99 m.

Dans le second type d'application, le groupement potentiellement effecteur pourra fixer un isotope émetteur de rayonnement ionisant, par exemple β notamment le Rhénium 186 ou 188 en vue d'une radio-thérapie.

Des chaînes de connexion préférées sont celles qui permettent une séparation suffisante des haptènes, notamment une chaîne de longueur supérieure à 10 Angströms, et sont notamment de nature peptidique ; ce sont par exemple des chaînes constituées de 3 à 12 acides aminés et de préférence de 3 à 6 acides aminés.

Ces chaînes seront de préférence peu sensibles à l'hydrolyse une fois injectées dans l'organisme. On utilise avantageusement à titre de chaînes de connexion des aminoacides non naturels, de préférence de série D, en enchaînement peptidique.

Pour certaines applications particulières, les chaînes de connexion ci-dessus décrites comprennent un groupement phénol et on retient tout particulièrement les chaînes de connexion renfermant de la tyrosine ou constituées de tyrosine.

Parmi les dérivés objets de la présente invention, l'on retient également ceux dont les haptènes sont des 3-[2-(4-imidazolyl)ethylaminocarbonyl]propionyl-glycyl (Histamine-Succinyl-Glycyl), qui sont couplés à une diamine phénolée, notamment couplés à un peptide renfermant de la tyrosine.

Parmi les dérivés selon la présente invention, on retient tout particulièrement les dérivés suivants : le S-acétyl-mercaptoacétyl-Glycyl-Glycyl-Glycyl-N-ε-(Histamine-Succinyl-Glycine)-Lysyl-D-Tyrosyl-N-ε-(Histamine-Succinyl-Glycine)-Lysinamide et le mercaptoacétyl Glycyl-Glycyl-Glycyl-N-ε-(Histamine-Succinyl-Glycine)-Lysyl-D-Tyrosyl-N-ε-(Histamine-Succinyl-Glycine)-Lysinamide.

La présente demande a également pour objet un procédé de préparation des dérivés de formule (I) ci-dessus ainsi que leurs sels, caractérisés en ce que l'on fait réagir un dérivé thiométhylé de formule (II)

R-S-CH₂-CO-M (II)

dans laquelle M représente un groupement libérable permettant le couplage d'un carbonyle sur une amine, de préférence un ester activé utilisable en synthèse peptidique, plus particulièrement un ester de N-hydroxysuccinimide, et R représente un radical R'CO dans lequel R' représente un alkyle renfermant 1 à 6 atomes de carbone, de préférence de 1 à 3 atomes de carbone ou un radical aryle, notamment phényl, avec un dérivé peptidique de formule (III) 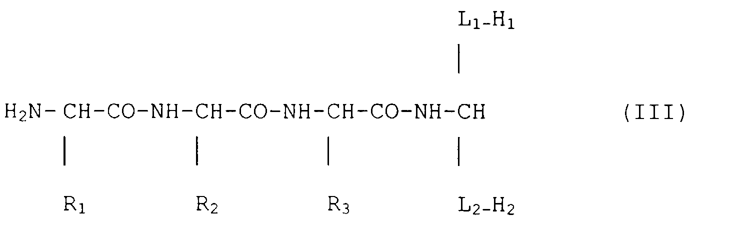 dans laquelle R₁, R₂, R₃, L₁, L₂, H₁ et H₂ ont la signification déjà indiquée pour obtenir le produit de formule (I) attendu que l'on isole ou salifie, si désiré.

Les dérivés ci-dessus décrits possèdent de remarquables propriétés grâce aux deux groupements hapténiques hydrophiles et à leur groupement peptidique effecteur. En présence de quantités convenables d'anticorps bispécifique, ils peuvent se lier rapidement, très spécifiquement et solidement à leur cible . Leur bruit de fond, c'est à dire leur localisation indésirable est très faible. Ils peuvent de plus lier des isotopes à durée de vie courte. Leur action in vivo est possible grâce à leur exceptionnelle rapidité de localisation permettant leur mise en oeuvre malgré la brièveté de la demi-vie des isotopes du Technétium et du Rhénium utilisables.

C'est pourquoi la présente demande a également pour objet l'application des dérivés tels que définis ci-dessus au diagnostic et à la thérapeutique.

Pour ces applications, on pourra se reporter notamment aux demandes de brevet européen EP-A-0 263 046 ainsi que EP-A-0 419 387.

En vue de leur utilisation diagnostique, les dérivés selon la présente invention peuvent être par exemple injectés par voie intraveineuse à l'homme ou à l'animal.

En même temps ou avant l'injection de la sonde, par exemple quelques minutes jusqu'à quelques heures avant (dans ce cas de préférence de 12 à 96 heures), on aura injecté un conjugué anticorps à double spécificité composé d'un anticorps notamment monoclonal ou fragment d'anticorps également de préférence monoclonal spécifique pour une cellule ou un composant normal ou pathologique d'un être vivant en particulier de l'homme, couplé à un anticorps, notamment monoclonal ou fragment d'anticorps de préférence spécifique pour le haptène considéré, tel que décrit ci-dessus en tant que produit (a), par exemple à la dose de 0,1 mg à 1 g selon la sonde utilisée et de préférence de 1 à 100 mg chez l'homme adulte.

Les conjugués anticorps peuvent être préparés par exemple comme décrit par Le Doussal et al dans Cancer Research 50, 3445-3452, June 1, 1990. Ils peuvent également être préparés par d'autres méthodes qui induisent un couplage chimique stable entre les deux anticorps ou fragments d'anticorps comme enseigné par exemple par Rodrigues et collaborateurs (International Journal of Cancer, supplement 7, 45-50 (1992)). Les produits obtenus par hybridation cellulaire entre cellules produisant chacun des deux anticorps ou par recombinaison génétique et expression dans des cellules prokaryotes ou eukaryotes sont également convenables dans la mesure où ils présentent au moins un site de liaison pour la cible et au moins un site de liaison pour le haptène.

Le dérivé a) peut être utilisé seul ou en mélange (cocktail), dans ce dernier cas chacun des dérivés du mélange ayant une spécificité particulière pour des cellules ou constituants de l'organisme normaux ou pathologiques différents et une spécificité pour le même haptène.

On procède après l'injection des dérivés selon l'invention à la mesure ou la détection grâce aux appareils biens connus dans l'état de la technique, par exemple pour la scintigraphie.

Dans le cas d'un diagnostic isotopique on aura par exemple apporté une activité de 0,1 à 100 mCi et plus particulièrement de 3 à 30mCi.

De même, on peut procéder au traitement radio-immunothérapeutique localisé d'une affection, par exemple tumorale, en utilisant une sonde selon la présente invention, possédant comme effecteur une isotope du Rhénium approprié.

Le taux sera alors limité par la toxicité secondaire du principe actif sur les organes normaux et en particulier sur la moelle osseuse.

Pour une application en radio-immunothérapie, une dose préférée sera comprise par exemple entre 50 mCi et 1 Ci.

Compte tenu de l'utilisation particulière des dérivés selon la présente invention, la présente demande a également pour objet les kits diagnostiques ou thérapeutiques, caractérisés en ce qu'ils renferment au moins un des dérivés selon la présente invention, tels que définis ci-dessus et un anticorps ou un fragment d'anticorps, notamment monoclonal reconnaissant un type cellulaire ou un tissu particulier, conjugué à un deuxième anticorps ou fragment d'anticorps reconnaissant un groupement hapténique du dit dérivé.

La présente demande a enfin pour objet les réactifs immunologiques et les kits comprenant un conjugué, composé d'un anticorps ou fragment d'anticorps notamment monoclonal ayant une affinité pour un type cellulaire particulier ou un constituant normal ou pathologique de l'organisme particulier, couplé à un anticorps ou fragment d'anticorps notamment monoclonal ayant une affinité pour un haptène donné, et une molécule synthétique, comprenant au moins deux haptènes correspondant au conjugué ci-dessus et au moins un site apte au marquage radioactif, liés de manière covalente, caractérisées en ce que la molécule synthétique est un dérivé ci-dessus défini.

Les dérivés de formule I peuvent être conservés lyophilisés, seuls ou en présence d'un chélatant intermédiaire tel que acétate, glycine, citrate, malonate et notamment tartrate, de préférence désoxygéné, et de chlorure stanneux, de préférence déshydraté.

Lorsque l'on parle de conjugué d'anticorps, on ne préjuge pas d'une méthode de préparation quelconque en ce sens que l'on vise aussi des anticorps bi-spécifiques encore appelés recombinants, quadromes ou polydomes, par exemple.

Les exemples qui suivent illustrent la présente invention sans toutefois la limiter.

### EXEMPLE 1 S-acétyl-mercaptoacétyl-Glycyl-Glycyl-Glycyl-N-E-(Histamine-Succinyl-Glycine)-Lysyl-D-Tyrosyl-N-ε-(Histamine-Succinyl-Glycine)-Lysinamide.

### Stade A :

### Préparation du Glycyl-Glycyl-Glycyl-N-ε-(Histamine-Succinyl-Glycine)-Lysyl-D-Tyrosyl-N-ε-(Histamine-Succinyl-Glycine)-Lysinamide.

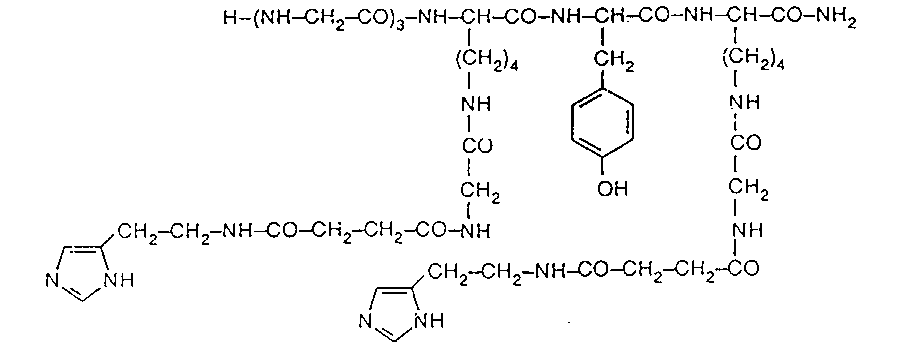

Ce peptide a été synthétisé par la technique de synthèse en phase solide.
- Toutes les étapes décrites ont lieu en phase solide.
- 1 g de résine p-méthyl benzhydrylamine (0,63 mmol NH₂) a été traitée par de la di-isopropyl-éthylamine (DIEA) à 5% dans du dichlorométhane (DCM) 3 fois 2 minutes. Puis les acides aminés suivants ont été couplés successivement : N-α-tBoc-N-ε-Fmoc-Lysine,N-α-tBoc-O-2, 6-dichlorobenzyl-D-tyrosine, N-α-tBoc-N-ε-Fmoc-Lysine, N-α-tBoc-Glycine, N-α-tBoc-Glycine, N-α-tBoc-Glycine (tBoc : tert-butyloxycarbonyle, Fmoc : fluorénylmethyloxycarbonyle). Les étapes suivantes ont été répétées pour le couplage de chaque acide aminé :

- Couplage : 1,57 mmol d'acide aminé protégé est ajouté à 1,57 mmol de dicyclohexylcarbodiimide (DCC) et 1,57 mmol de 1-hydroxybenzotriazole (HOBt), en solution dans un mélange de diméthylformamide (DMF)/DCM, 1/1, v/v.
   Après une heure (1/2 heure dans le cas des N-α-tBoc-Glycines), la résine est lavée avec du DMF (3 fois) et du DCM (3 fois). L'absence de fonctions NH₂ libres sur la résine est vérifiée par le test de Kaiser. Si nécessaire le couplage est recommencé dans les mêmes conditions. Si après deux couplages successifs le test de Kaiser révèle la présence de fonctions NH₂ libres, la résine est alors acétylée par 2,5 mmol d'anhydride acétique dans du DMF en présence de 2,5 mmol de DIEA.
- Déprotection de la fonction α-NH₂. La déprotection du groupement α-NH₂ est ensuite effectuée par de l'acide trifluoroacétique dans du DCM (50%), pendant 2 minutes puis 20 minutes, puis après deux lavages au DCM la résine est traitée par de la DIEA (5% dans du DCM), 2 fois 2 minutes, après 3 lavages au DMF le couplage suivant est effectué.
   Après incorporation de la tBoc-Glycine N-terminale la protection de la fonction α-NH₂ de la glycine est conservée et les groupements Fmoc protégeant les groupements ε-NH₂ des lysines sont clivés en présence d'un mélange de piperidine / DMF, 1/1, v/v, pendant 2 minutes puis 40 minutes. Après 3 lavages par le DCM et 3 lavages par le DMF, 3,15 mmol de N-α-Fmoc glycine sont ajoutées en présence de 3,15 mmol de DCC et de 3,15 mmol d'HOBt, dans un mélange DMF / DCM, 1/1, v/v. Après 1/2 heure le couplage est contrôlé par le test de Kaiser, et renouvelé dans les mêmes conditions si nécessaire. Puis le groupement Fmoc protégeant la fonction α-NH₂ de la glycine est clivé de la même façon que précédemment.
   Après lavage (3 fois par du DCM et 3 fois par du DMF) de l'anhydride succinique (5 mmol) est ajouté en présence de 5 mmol de di-isopropyléthylamine (DIEA) après 1 heure la réaction est contrôlée par le test de Kaiser et le couplage renouvelé dans les mêmes conditions si nécessaire. Après lavage (3 fois par du DCM et 3 fois par du DMF) du dichlorhydrate d'histamine (5 mmol) est ajouté en présence de 25 mmol de DIEA, de 5 mmol d'hexafluorophosphate de benzotriazol-1-yl-oxy-tris-(diméthylamino)phosphonium (BOP) et 5 mmol de HOBt en solution dans un mélange diméthylsulfoxyde (DMSO)/DMF, 5/2, v/v.
   Après deux heures de réaction la résine est lavée au DMSO (3 fois) puis au DMF (3 fois) et le couplage est répété dans les mêmes conditions. Après 12 heures de réaction la résine est lavée comme précédemment, puis séchée sous pression réduite. Le peptide est alors clivé de la résine par l'acide fluorhydrique liquide (HF) : pour 600 mg de peptidyl résine, 750 µl de para crésol, 375 µl de diméthylsulfure sont ajoutés et 6 ml d'acide fluorhydrique sont distillés dans le réacteur. La réaction est maintenue à 4°C 1 heure sous agitation puis le HF est éliminé par distillation. La résine est alors lavée à l'éther puis le peptide est élué par de l'acide acétique 0,1 M. Le peptide est alors purifié par chromatographie de perméation de gel sur une colonne de TSK HW40.
   La pureté est vérifiée par HPLC sur une colonne de phase inverse (C18) éluée par un gradient de solvants (A : acide trifluoroacétique dans l'eau à 0,5‰, B : Acétonitrile).

L'identité du produit a été vérifiée par spectrométrie de masse : (M+H)⁺ observé : 1110,1 ; calculé : 1109,2.

### Stade B :

### Préparation du S-acétyl-mercaptoacétyl-Glycyl-Glycyl-Glycyl-N-ε-(Histamine-Succinyl-Glycine)-Lysyl-D-Tyrosyl-N-ε-(Histamine-Succinyl-Glycine)-Lysinamide.

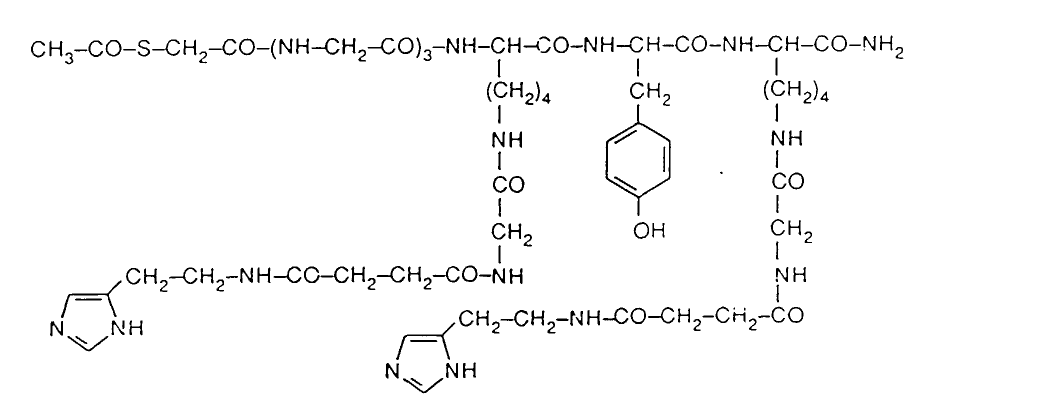

A 32,5 µmol de produit du stade A en solution dans 12 ml de tampon phosphate (pH 7,4 ; 50 mM) sont ajoutés doucement sous agitation 81 µmol de l'ester de N-hydroxysuccinimide de l'acide S-acétyl-mercaptoacétique en solution dans 140 µl de DMSO. La réaction est laissée sous agitation 3/4 d'heure à température ambiante. Le produit obtenu est purifié par HPLC sur une colonne de phase inverse (C18), et élué par un gradient de solvants (A : acide trifluoroacétique dans l'eau à 0,5 ‰, B : Acétonitrile).

L'identité a été vérifiée par spectrométrie de masse : (M+H)⁺ observé : 1225,8 ; calculé : 1225,3.

### EXEMPLE 2 Mercaptoacétyl-Glycyl-Glycyl-Glycyl-N-ε-(Histamine-Succinyl-Glycine)-Lysyl-D-Tyrosyl-N-ε-(Histamine-Succinyl-Glycine)-Lysinamide.

A 0,18 µmol du dérivé obtenu à l'exemple 1, en solution dans 1 ml de tampon phosphate (pH 7,4; 50 mM) sont ajoutés 0,72 µmol de chlorhydrate d'hydroxylamine en solution dans 1 ml de tampon phosphate (50 mM ; ajusté à pH 7,4). La réaction est maintenue sous agitation une heure à température ambiante, puis 5 µl de mercaptoéthanol sont ajoutés et la réaction est maintenue 1 heure à la température ambiante. Le peptide est alors purifié par chromatographie haute performance sur une colonne de phase inverse (C₁₈), et élué par un gradient de solvants (A : acide trifluoroacétique dans l'eau à 0,5 ‰, B : acétonitrile). L'identité à été vérifiée par spectrométrie de masse : (M+H)⁺ observé : 1183,8 ; calculé: 1183,3.

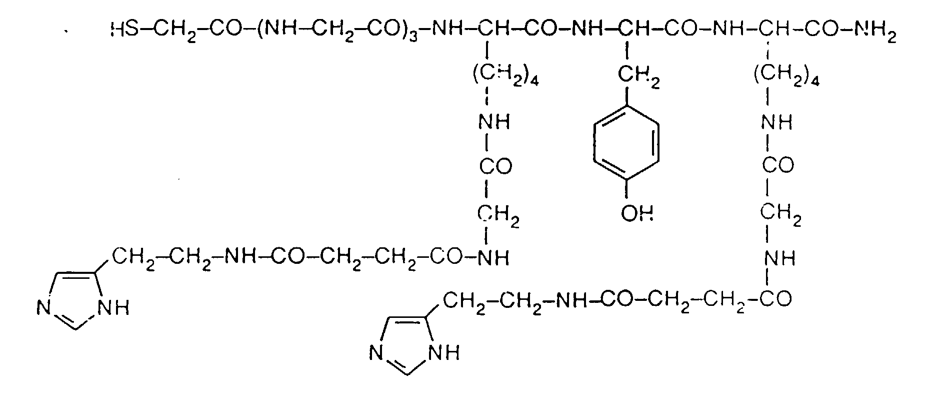

### EXEMPLE 3 Synthèse de ^{99m}Tc mercaptoacétyl-Glycyl-Glycyl-Glycyl-N-ε-(Histamine-Succinyl-Glycine)-Lysyl-D-Tyrosyl-N-ε-(Histamine-Succinyl-Glycine)-Lysinamide.

L'éluat d'un générateur de ^{99m}Tc (Elumatic III, Cis bicinternational) est dilué avec du soluté physiologique de façon à avoir 10mCi/ml. 100 µl (1mCi) sont mélangés à 0,1 nmol de produit de l'exemple 1 en présence de 2 nmol de tartrate disodique, de 5 nmol de chlorure stanneux et de 10µmol d'HEPES tamponné à pH 8. Le mélange est agité et chauffé au bain marie à 100 °C pendant 10 mn, puis refroidi sous l'eau froide pour obtenir le produit marqué attendu.

### Détermination du rendement de marquage :

Un échantillon du produit ci-dessus est dilué dans du tampon phosphate salin pH 7,4 contenant 1 % d'albumine bovine de façon à avoir environ 100000 cpm dans 1 ml. 300 pl de la dilution sont incubés 1 heure sous agitation à température ambiante dans des tubes recouverts d'anticorps 679.1MC7 (1 µg/l ml) dirigé contre l'haptène glycyl-succinylhistamine. La radioactivité totale présente dans chaque tube est déterminée, puis les tubes sont vidés et lavés et la radioactivité liée est déterminée. Le rendement de marquage (92 à 97 %) est évalué par le rapport radioactivité liée / radioactivité totale.

### LIAISON DE LA SONDE MARQUEE AU ^{99m}Tc SUR DES CELLULES CIBLES IN VITRO

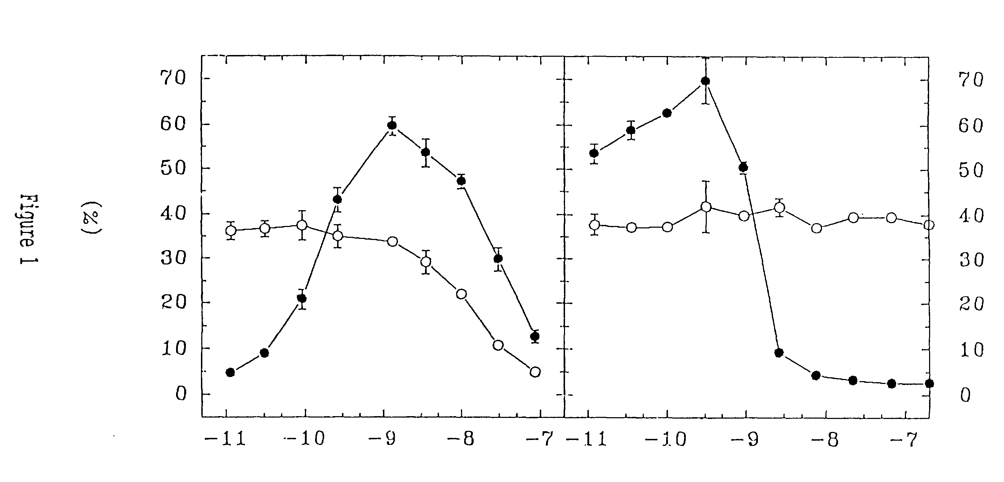

Des cellules LS-174 T (7.10⁶ cellules/ml), cellules d'origine humaine connues pour exprimer à leur surface l'antigène carcino-embryonnaire, sont incubées à 4° C pendant 2 heures dans des tubes contenant un tampon phosphate isotonique à pH 7,3. Dans ces tubes, ont été ajoutés à des concentrations variables le conjugué bispécifique approprié (anti-antigène carcino-embryonnaire/anti-histamine-succinyl-glycine), marqué à l'iode-125 suivant une des techniques bien connues et la sonde, marquée au ^{99m}Tc comme décrit ci-dessus. Ces concentrations sont calculées pour couvrir un domaine s'étendant de concentrations très inférieures à très supérieures à la concentration de l'antigène cible dans le mélange. A la fin de cette incubation, 100 µl de mélange sont transférés en triplicates dans des tubes contenant un mélange de phthalates. Les tubes sont centrifugés 30 secondes à environ 5000 g. Les cellules, plus denses que les phthalates, forment un culot alors que le surnageant reste à la surface. On peut alors compter séparément les activités (¹²⁵I et ^{99m}Tc) liées aux cellules et les activités non liées et en calculer les rapports. Les résultats sont regroupés en deux panneaux sur la figure suivante pour faire apparaître la liaison aux cellules-cibles de la sonde et de l'anticorps bispécifiques soit à la concentration constante de sonde (panneau de gauche), soit à concentration constante d'anticorps bispécifique (panneau de droite). Les résultats sont exprimés en pourcentage de sonde (points noirs) ou d'anticorps bispécifique (points blancs) lié par rapport à leur quantité totale, en fonction des concentrations molaires d'anticorps bispécifique (tableau de gauche) ou de sonde (tableau de droite) ; par exemple -9 représente une concentration 10⁻⁹ M. On voit que la sonde, révélée par son activité ^{99m}Tc, se lie très fortement aux cellules cibles, jusqu'à près de 70 %, pour des concentrations nanomolaires d'anticorps bi-spécifique.

### ETUDE PHARMACOLOGIQUE

Lorsque la sonde marquée au ^{99m}Tc (13 µCi, 2 pmol) est injectée seule à des souris, on observe une élimination très rapide de l'activité, principalement dans les urines. Par exemple, 6 heures après injection, on ne retrouve que moins de 0,15 % de la dose injectée par ml de plasma, 0,25 % par gramme de tumeur, 1 % par gramme de foie et 0,2 % par gramme de rate. Ceci confirme que la sonde de l'exemple 3, marquée au technétium, présente bien la forte clearance nécessaire pour les applications diagnostiques et thérapeutiques envisagées.
1) Marquage in vivo du foie des souris à l'aide d'anticorps 679.1MC7 et du dérivé de l'exemple 3 (I marqué à TC).
   La liaison du dérivé de l'exemple 3 aux cellules hépatiques s'effectue par l'intermédiaire de sa liaison au site Ac de l'anticorps 679.1MC7, lui même lié par son fragment F(c) à ces cellules. Des souris ayant reçu 2µg d'anticorps 679.1MC7 24 heures plus tôt sont injectées par voie intraveineuse avec 100 µCi (15 pmol) de dérivé de l'exemple 3. Les images sont réalisées avec une caméra Sopha Medical Gammatome 2 équipée avec un collimateur haute résolution de moyenne énergie, puis les animaux sont sacrifiés et la radioactivité présente dans le foie est déterminée à l'aide d'un compteur gamma. Le foie est apparent en scintigraphie quatre heures après l'injection. Les animaux sont alors sacrifiés et le foie est prélevé et sa radioactivité déterminée : 54 ± 6 % de la dose injectée est localisée dans le foie.
   Les sondes selon la présente invention peuvent donc servir au marquage d'un tissu auquel est lié un anticorps.
2) Marquage avec le produit de l'exemple 3 des lymphocytes de souris BALB/c qui expriment l'antigène Lyb 8.2 pour visualiser la rate de ces animaux.
   100 µCi de dérivé de l'exemple 3 sont injectés à une souris ayant reçu quelques minutes plus tôt 1µg de conjugué anticorps CY34-679.1MC7 (anti-lyb8.2-anti-Histamine-Succinyl-Glycine). Les images sont enregistrées comme indiqué dans l'exemple précédent. La rate est apparente en scintigraphie quatre heures après l'injection. Les animaux sont ensuite sacrifiés et la rate est prélevée : 43 ± 3 % de la dose injectée est localisée dans la rate.
   Par l'intermédiaire d'une sonde selon la présente invention à double spécificité tissu-haptène, on peut fixer la sonde sur le tissu, même en cas d'administration quasiment simultanée d'anticorps à double spécificité et de sonde selon l'invention et ainsi procéder à la visualisation du tissu concerné.
3) Ciblage du produit de l'exemple 3 dans des tumeurs colorectales humaines (LS174) greffées à des souris nudes.
   Des cellules de tumeurs colorectales humaines (cellules LS 174, 3.10⁶) sont injectées par voie sous cutanée à des souris nudes. Trois semaines plus tard des tumeurs de 0,3 à 1,2 g sont obtenues. Les souris reçoivent alors 2µg de conjugué anticorps F6-679.1MC7 (antiCEA-anti-Histamine-Succinyl-Glycine). Vingt quatre heures plus tard les souris reçoivent 6,7µCi (1pmol) de dérivé de l'exemple 3. Six heures plus tard les images sont enregistrées comme indiqué dans l'exemple précédent puis les animaux sacrifiés. Les tumeurs sont visibles dès trois heures après l'injection du dérivé de l'exemple 3. Six heures après injection de la sonde, on observe une localisation de la radioactivité de 9,8 ± 3,7 % / g de tumeur et le rapport de marquage tumeur/plasma est de 2.0 + 0.5.
   Il est donc possible de détecter des tumeurs humaines grâce à des anticorps à double spécificité correspondant à la tumeur et aux haptènes porté par la sonde, et à une sonde selon l'invention.
   La radioactivité excrétée dans les urines conserve environ 90 % d'immunoréactivité vis-à-vis d'un anticorps reconnaissant le haptène histamine-succinyl-glycine.

### EXEMPLE 4 Tolérance des sondes chez l'animal.

Des doses correspondant à 10 nanomol, soit environ 10000 fois plus que la dose qui est nécessaire pour une radio-immunoscintigraphie, du composé 3 ont été administrées à des souris BALB/c. Ces souris ont ensuite été observées pendant 16 jours dans des conditions normales d'élevage. On n'a constaté aucun signe de toxicité et la prise de poids de ces animaux n'a pas été significativement différente de celle d'un lot d'animaux témoins.

## Revendications

1. Dérivés comprenant deux haptènes hydrophiles et un groupement potentiellement effecteur caractérisés en ce qu'ils répondent à la formule générale 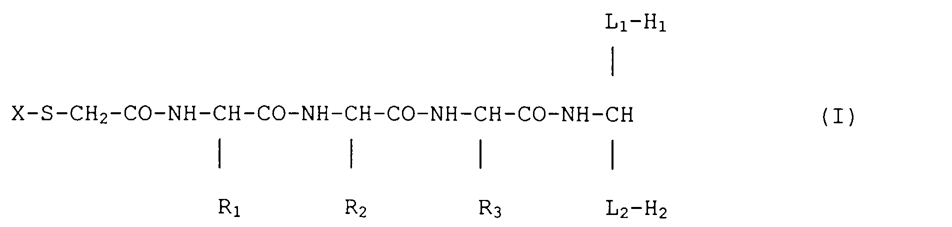 ainsi que leurs sels, dans laquelle X représente un atome d'hydrogène ou un groupe protecteur utilisable pour protéger une fonction thiol contre l'oxydation R₁, R₂ et R₃ représentent chacun une chaîne latérale d'un acide aminé, L₃ et L₂ représentent chacun une liaison stable permettant la fixation simultanée sur le même dérivé d'au moins 2 anticorps spécifiques sur les haptènes, la distance séparant les haptènes étant supérieure à 10 Angströms et H₁ et H₂ représentent des haptènes hydrophiles.

2. Dérivés selon la revendication 1, caractérisés en ce que les haptènes hydrophiles ont un coefficient de partition eau-butanol supérieur à 1.

3. Dérivés selon la revendication 1 ou 2, caractérisés en ce que les haptènes ont une constante de dissociation inférieure ou égale à 10⁻⁵ M avec un anticorps qui leur est spécifique.

4. Dérivés selon l'une quelconque des revendications 1 à 3, caractérisés en ce que les haptènes sont des dérivés d'amines, d'acides ou d'acides aminés polaires liés de façon covalente par des liaisons amide.

5. Dérivés selon l'une quelconque des revendications 1 à 4, caractérisés en ce que les haptènes sont fixés au groupement potentiellement effecteur par l'intermédiaire d'une chaîne de connexion qui est un peptide.

6. Dérivés selon l'une quelconque des revendications 4 ou 5, caractérisés en ce que les haptènes sont des groupements Histamine-Succinyl-Glycine.

7. L'un des dérivés dont les noms suivent:
- le S-acétyl-mercaptoacétyl-Glycyl-Glycyl-Glycyl-N-ε-(Histamine-Succinyl-Glycine)-Lysyl-D-Tyrosyl-N-ε-(Histamine-Succinyl-Glycine) -Lysinamide,
- le mercaptoacétyl-Glycyl-Glycyl-Glycyl-N-ε-(Histamine-Succinyl-Glycine)-Lysyl-D-Tyrosyl-N-ε-(Histamine-Succinyl-Glycine)-Lysinamide,ou l'un de leurs sels.

8. Procédé de préparation des dérivés de formule (I) ci-dessus ainsi que leurs sels, caractérisés en ce que l'on fait réagir un dérivé thiométhylé de formule (II)
R-S-CH₂-CO-M (II)
dans laquelle M représente un groupement hydrolysable permettant le couplage d'un carbonyle sur une amine, de préférence un ester activé utilisable en synthèse peptidique, plus particulièrement le N-hydroxysuccinimide, et R représente un radical R'CO dans lequel R' représente un alkyle renfermant 1 à 6 atomes de carbone, de préférence de 1 à 3 atomes de carbone ou un radical aryle, notamment phényl, avec un dérivé peptidique de formule (III) 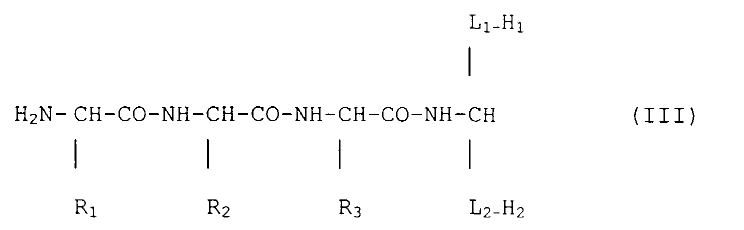 dans laquelle R₁, R₂, R₃, L₁, L₂, H₁ et H₂ ont la signification déjà indiquée pour obtenir le produit de formule (I) attendu que l'on isole ou salifie, si désiré.

9. Application des dérivés tels que définis à l'une quelconque des revendications 1 à 7 au diagnostic ou à la thérapeutique.

10. Kits diagnostiques ou thérapeutiques, caractérisés en ce qu'ils renferment au moins un des dérivés selon l'une quelconque des revendications 1 à 7 et un anticorps ou fragment d'anticorps, notamment monoclonal reconnaissant un type cellulaire ou un tissu particulier, conjugué à un deuxième anticorps ou fragment d'anticorps reconnaissant un groupement hapténique du dit dérivé.

11. Réactifs immunologiques comprenant un conjugué composé d'un anticorps ou fragment d'anticorps notamment monoclonal ayant une affinité pour un type cellulaire particulier ou un constituant normal ou pathologique de l'organisme particulier, couplé à un anticorps ou fragment d'anticorps notamment monoclonal ayant une affinité pour un haptène donné, et une molécule synthétique comprenant au moins deux haptènes correspondant au conjugué ci-dessus et au moins un site, apte au marquage radioactif ou à fixer un principe actif, liés de manière covalente, caractérisés en ce que la molécule synthétique est un dérivé défini à l'une des revendications 1 à 7.

## Claims

1. Derivatives comprising two hydrophilic haptens and a potentially effector group characterized in that they correspond to general formula 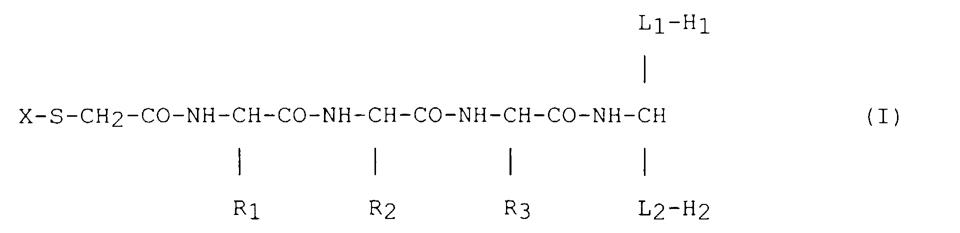 as well as their salts, in which X represents a hydrogen atom or a protective group which can be used to protect a thiol function from oxidation, R₁, R₂ and R₃ each represent a side chain of an amino acid, L₁ and L₂ each represent a stable bond allowing the simultaneous fixation on the same derivative of at least 2 specific antibodies on the haptens, the distance separating the haptens being greater than 10 Angstroms and H₁ and H₂ represent hydrophilic haptens.

2. Derivatives according to claim 1, characterized in that the hydrophilic haptens have a partition coefficient water-butanol greater than 1.

3. Derivatives according to claim 1 or 2, characterized in that the haptens have a dissociation constant less than or equal to 10⁻⁵ M with an antibody which is specific for them.

4. Derivatives according to any one of claims 1 to 3, characterized in that the haptens are derivatives of amines, acids or polar amino acids linked in a covalent fashion by amide bonds.

5. Derivatives according to any one of claims 1 to 4, characterized in that the haptens are fixed to the potentially effector group by means of a connecting chain which is a peptide.

6. Derivatives according to any one of claims 4 or 5, characterized in that the haptens are histamine-succinyl-glycine groups.

7. One of the derivatives whose names follow:
- S-acetyl-mercaptoacetyl-glycyl-glycyl-glycyl-N-ε-(histamine-succinyl-glycine)-lysyl-D-tyrosyl-N-ε-(histamine-succinyl-glycine)-lysinamide,
- mercaptoacetyl-glycyl-glycyl-glycyl-N-ε-(histamine-succinyl-glycine)-lysyl-D-tyrosyl-N-ε-(histamine-succinyl-glycine)-lysinamide, or one of their salts.

8. Process for the preparation of the derivatives of formula (I) above as well as their salts, characterized in that a thiomethylated derivative of formula (II)
R-S-CH₂-CO-M (II)
in which M represents a hydrolyzable group allowing the coupling of a carbonyl on an amine, preferably an activated ester such as those used in peptide synthesis, more particularly the N-hydroxysuccinimide and R represent an R'CO radical in which R' represents an alkyl radical containing 1 to 6 carbon atoms, preferably 1 to 3 carbon atoms or an aryl radical, in particular phenyl, is reacted with a peptidic derivative of formula (III) 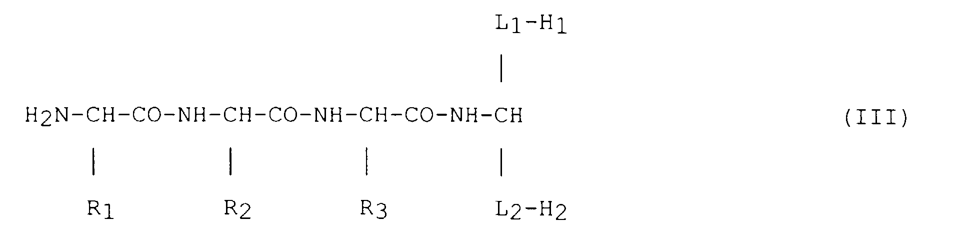 in which R₁, R₂, R₃, L₁, L₂, H₁ and H₂ have the meaning already indicated to obtain the expected product of formula (I) which is isolated or salified, if desired.

9. Use of the derivatives as defined in any one of claims 1 to 7 in diagnostics or therapeutics.

10. Diagnostic or therapeutic kits characterized in that they contain at least one of the derivatives according to any one of claims 1 to 7 and an antibody or a fragment of an antibody, in particular monoclonal, which recognises a particular cell type or tissue, conjugated to a second antibody or a fragment of an antibody which recognises a hapten group of said derivative.

11. Immunological reagents comprising a conjugate composed of an antibody or a fragment of an antibody, in particular monoclonal, having an affinity for a particular cell type or normal or pathological component of the particular organism, coupled to an antibody or a fragment of an antibody, in particular monoclonal, having an affinity for a given hapten, and a synthetic molecule comprising at least two haptens corresponding to the above conjugate and at least one site, suitable for radioactive labelling or to fix an active principle, linked in a covalent manner, characterized in that the synthetic molecule is a derivative defined in any one of claims 1 to 7.

## Patentansprüche

1. Derivate umfassend zwei hydrophile Haptene und eine potentielle Effektorgruppe, dadurch gekennzeichnet, dass sie der allgemeinen Formel 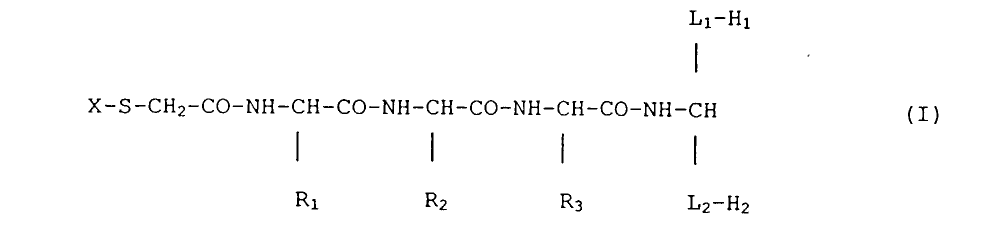 entsprechen, sowie ihre Salze, worin X ein Wasserstoffatom oder eine zum Schutz einer Thiolfunktion gegen Oxidation einsetzbare Schutzgruppe darstellt, R1, R2 und R3 jeweils eine Seitenkette einer Aminosäure darstellen, L1 und L2 jeweils für eine stabile Bindung stehen, die die gleichzeitige Fixierung mindestens zweier spezifischer Antikörper an den Haptenen an ein- und dasselbe Derivat gestattet, wobei der die Haptene trennende Abstand größer als 10 Angström ist, und H1 und H2 hydrophile Haptene darstellen.

2. Derivate nach Anspruch 1, dadurch gekennzeichnet, dass die hydrophilen Haptene einen Verteilungskoeffizienten Wasser-Butanol von größer als 1 aufweisen.

3. Derivate nach Anspruch 1 oder 1, dadurch gekennzeichnet, dass die Haptene eine Dissoziationskonstante von unter oder gleich 10-5 M mit einem Antikörper aufweisen, der spezifisch für sie ist.

4. Derivate nach einem der Ansprüche 1 bis 3, dadurch gekennzeichnet, dass die Haptene polare Amin-, Säure- oder Aminosäurederivate sind, die auf kovalente Weise mittels Amidbindungen gebunden sind.

5. Derivate nach einem der Ansprüche 1 bis 4, dadurch gekennzeichnet, dass die Haptene mittels einer Verbindungskette in Form eines Peptids an der potentiellen Effektorgruppe fixiert sind.

6. Derivate nach einem der Ansprüche 4 oder 5, dadurch gekennzeichnet, dass die Haptene Histamin-Succinyl-Glycin-Gruppen sind.

7. Derivat mit folgender Bezeichnung:
- S-Acetylmercaptoacetyl-Glycyl-Glycyl-Glycl-N-e-(Histamin-Succinyl-Glycin)-Lysyl-D-Tyrosyl-N-e-(Histamin-Succinyl-Glycin)-Lysinamid oder
- Mercaptoacetyl-Glycyl-Glycyl-Glycl-N-e-(Histamin-Succinyl-Glycin)-Lysyl-D-Tyrosyl-N-e-(Histamin-Succinyl-Glycin)-Lysinamid
oder eines ihrer Salze.

8. Verfahren zur Herstellung der Derivate der obigen Formel (I) sowie ihrer Salze, dadurch gekennzeichnet, dass ein Thiomethylderivat der Formel (II)
R-S-CH2-CO-M (II)
worin M eine die Kopplung eines Carbonyls an ein Amin ermöglichende hydrolysierbare Gruppe, vorzugsweise einen in der Peptidsynthese einsetzbaren aktivierten Ester, insbesondere N-Hydroxysuccinimid, darstellt und R eine Gruppe R'CO ist, in der R' ein Alkyl mit 1 bis 6 Kohlenstoffatomen, vorzugsweise 1 bis 3 Kohlenstoffatomen, oder eine Arylgruppe, insbesondere Phenyl, darstellt, mit einem Peptidderivat der Formel (III) 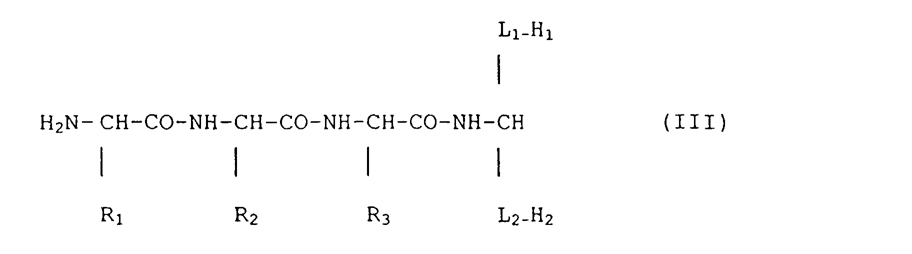 worin R1, R2, R3, L1, L2, H1 und H2 die bereits angegebene Bedeutung haben, zur Umsetzung gebracht wird, um das erwartete Produkt der Formel (I) zu ergeben, das gewünschtenfalls isoliert oder in ein Salz übergeführt wird.

9. Anwendung der Derivate nach einem der Ansprüche 1 bis 7 in der Diagnose oder in der Therapie.

10. Diagnose- oder therapeutische Kits, dadurch gekennzeichnet, dass sie mindestens ein Derivat nach einem der Ansprüche 1 bis 7 und einen Antikörper oder ein Antikörperfragment, insbesondere einen monoklonalen Antikörper oder ein monoklonales Antikörperfragment, der bzw. das einen speziellen Zelltyp oder ein spezielles Gewebe erkennt, konjugiert an einen zweiten Antikörper oder ein zweites Antikörperfragment, der bzw. das eine Haptengruppe des Derivats erkennt, enthalten.

11. Immunologische Reagenzien, umfassend ein Konjugat gebildet durch einen Antikörper oder ein Antikörperfragment, insbesondere einen monoklonalen Antikörper oder ein monoklonales Antikörperfragment mit einer Affinität für einen speziellen Zelltyp oder einen normalen oder pathologischen Bestandteil des speziellen Organismus, der bzw. das an einen Antikörper oder ein Antikörperfragment, insbesondere einen monoklonalen Antikörper oder ein monoklonales Antikörperfragment mit einer Affinität für ein bestimmtes Hapten gekoppelt ist, und ein synthetisches Molekül mit mindestens zwei dem obigen Konjugat entsprechenden Haptenen und mindestens einer für eine radioaktive Markierung oder die Fixierung eines Wirkstoffs geeigneten Stelle, die auf kovalente Weise gebunden sind, dadurch gekennzeichnet, dass das synthetische Molekül ein Derivat nach einem der Ansprüche 1 bis 7 ist.
